# EUROPEAN PATENT APPLICATION

(11) **EP 3 587 557 A1**
(43) Date of publication of application: **01.01.2020**
(21) Application number: 18747454.9
(22) Date of filing: 05.02.2018
(51) Int. Cl.: C12N 1/20, B09B 3/00, B09B 5/00, C12R 1/07

(54) **MICROORGANISM ISOLATED FROMTENEBRIO**

(30) Priority: 03.02.2017 KR 20170015553
(71) Applicant: Suh, Dong Eun, Yongin-si, Gyeonggi-do 17024 (KR)
(72) Inventor: CHENG, Jinhua, Yongin-si Gyeonggi-do 17057 (KR); SUH, Dong Eun, Yongin-si, Gyeonggi-do 17024 (KR)
(74) Representative: Eisenführ Speiser
(86) International application number: PCT/KR2018/001517
(87) International publication number: WO 2018/143750

(57) **Abstract**

The present invention provides a microorganism isolated from *Tenebrio molitor* larva and having plastic degradation activity. A microorganism having plastic degradation activity according to the present invention can selectively or entirely degrade at least one plastic selected from polyethylene terephthalate (PET), polyvinyl chloride (PVC), polystyrene (PS), polypropylene (PP), and polyethylene (PE) into low-molecular weight materials in an appropriate culture condition. Therefore, the microorganism having plastic degradation activity according to the present invention may be used in a pre-treatment process for plastic recyling.

## Description

### [Technical Field]

The present invention relates to a microorganism having plastic degradation activity and the use thereof, more specifically, to a microorganism isolated from *Tenebrio molitor* larva and capable of selectively or entirely degrading various types of plastic, and a method of degrading plastic using the same.

### [BackgroundArt]

Waste includes a variety of materials such as metals, glass, plastic, and the like. Among these materials, plastic accounts for a much larger part of both industrial waste and domestic waste than metals. Accordingly, if the value of waste plastic is economically converted, it can be seen that plastic exhibits a higher recycling value than metals. In addition, due to the depletion of a petroleum resource which is the main raw material of plastic, there is a growing need for recycling waste plastic.

When plastic is recycled, various types of plastic are collected all together and recycled. Usually, various types of plastic are used in one product and for this reason, when plastic is recycled individual plastic materials act as impurities against other plastic materials during the recycling, making it difficult to completely keep purity as a single plastic material. As a result, the quality of recycled plastic is degraded, and thus its economic value is estimated to be about 34% lower than un-recycled plastic. Accordingly, since it is difficult to economically separate mixed waste plastic included in domestic waste according to different material properties of each plastic kind, there is an urgent need to develop a technique for sorting waste plastic in a mixed state.

In general, the plastic recycling process is largely composed of a pretreatment process and a main-recycling process. In the pretreatment process, waste plastic in which various materials are mixed is separated and sorted according to property.

To separate and sort the mixed waste plastic according to a property, a wet-type floatation method has been commonly used. However, the wet-type floatation method requires a large amount of water, and even when the mixed waste plastic is subjected to wet-type floatation, about 2% other materials still remain mixed, and thus the quality of recycled plastic is degraded.

Particularly, polyvinyl chloride (PVC) is not easily separated through wet-type floatation and thus is likely to act as a foreign substance against other plastics. Therefore, the pre-removal technology of PVC is very important in the pretreatment process of the plastic recycling process.

Recently, a technique for disposing of plastic included in wastewater or waste using a microorganism has been developed. For example, Patent Document 1 (Korean Registered Patent No. 10-0513931) discloses *Microbacterium barkeri* LC (Accession (deposition) Number: KCCM 10507) and a method of biologically degrading polyvinyl alcohol using the same.

In addition, it was discovered in 2015 that mealworms can degrade polystyrene into reusable organic materials (Non-Patent Document 1). Mealworms are the larvae of *Tenebrio molitor* that is an insect belonging to the family Tenebrionidae. Since mealworms can be cultured, there is a growing interest in a technology for degrading plastic using them. However, nothing is known about the plastic degradation mechanism of mealworms, and thus commercialization has not been attempted.

### (Prior-Art Documents)

Patent Document 1: Korean Registered Patent No. 10-0513931

Non-Patent Document 1: Jordan, Rob. Stanford News Service, Retrieved 24 March 2016, [Plastic-eating worms may offer solution to mounting waste, Stanford researchers discover].

### [Disclosure]

### [Technical Problem]

The present invention has been made under the conventional technical background and is directed to providing a microorganism isolated from *Tenebrio molitor* larva and having plastic degradation activity, and a method of degrading plastic using the same.

### [Technical Solution]

One aspect of the present invention provides a microorganism isolated from *Tenebrio molitor* larva, selected from the group consisting of *Klebsiella oxytoca, Escherichia fergusonii,* and *Bacillus toyonensis* reborn (Accession (deposition) Number: KACC 81043BP), and having plastic degradation activity.

Another aspect of the present invention provides a method of degrading plastic, which includes culturing a microorganism having plastic degradation activity together with plastic-containing waste or waste plastic, wherein the microorganism having plastic degradation activity is isolated from *Tenebrio molitor* larva and is one or more selected from the group consisting of *Klebsiella oxytoca, Escherichia fergusonii,* and *Bacillus toyonensis* reborn (Accession (deposition) Number: KACC 81043BP).

A temperature and duration of the culture of the microorganism may be 25 °C to 40 °C and 10 days to 60 days, respectively.

Still another aspect of the present invention provides a method of degrading plastic, which includes reacting plastic-containing waste or waste plastic with an enzyme having plastic degradation activity, wherein the enzyme having plastic degradation activity is produced by a microorganism isolated from *Tenebrio molitor* larva and being one or more selected from the group consisting of *Klebsiella oxytoca, Escherichia fergusonii,* and *Bacillus toyonensis* reborn (Accession (deposition) Number: KACC 81043BP).

Yet another aspect of the present invention provides a culture solution of *Bacillus toyonensis* reborn (Accession (deposition) Number: KACC 81043BP) isolated from *Tenebrio molitor* larva and having plastic degradation activity.

In the above-described aspects, the plastic may be one or more selected from the group consisting of polyethylene terephthalate (PET), polyvinyl chloride (PVC), polystyrene (PS), polypropylene (PP), and polyethylene (PE).

### [Advantageous Effects]

A microorganism having plastic degradation activity according to the present invention can selectively or entirely degrade, under appropriate culture conditions, at least one plastic selected from polyethylene terephthalate (PET), polyvinyl chloride (PVC), polystyrene (PS), polypropylene (PP), and polyethylene (PE) into low-molecular-weight materials. Also, a microorganism having plastic degradation activity according to the present invention can be used in selective plastic degradation or in the pretreatment process for recycling plastic using selective degradation.

### [Description of Drawings]

FIG. 1 shows the result of evaluating the plastic degradation activity of a microorganism isolated from *Tenebrio molitor* larva using a solid medium.
FIG. 2 shows the gas chromatography analysis result of a culture solution of a control after 15-day culture in a polyvinyl chloride (PVC) degradation experiment using a liquid medium.
FIG. 3 shows the gas chromatography analysis result of a culture solution of a selected strain, *Escherichia fergusonii,* after 15-day culture in a PVC degradation experiment using a liquid medium.

### [Modes of the Invention]

According to one aspect of the present invention, there is provided a microorganism isolated from *Tenebrio molitor* larva, selected from *Klebsiella oxytoca, Escherichia fergusonii,* and *Bacillus toyonensis* reborn (Accession (deposition) Number: KACC 81043BP), and having plastic degradation activity.

The microorganism isolated from *Tenebrio molitor* larva has an activity of degrading at least one plastic selected from the group consisting of polyethylene terephthalate (PET), polyvinyl chloride (PVC), polystyrene (PS), polypropylene (PP), and polyethylene (PE).

Therefore, the microorganism of the present invention can selectively or entirely degrade, under appropriate culture conditions, the plastic into low-molecular-weight materials, and can be used in selective plastic degradation or in the pretreatment process for recycling plastic to enhance efficiency of plastic recycling, productivity, advantages resulting from selective degradation, and the like.

The degree of plastic degradation activity of the microorganism isolated from *Tenebrio molitor* larva may vary depending on a culture temperature, a culture duration, and the like.

Although a temperature and duration of culture are not particularly limited, for example, a culture temperature may be 25 °C to 40 °C, and a culture duration may be 10 days to 60 days. When the above-described culture conditions are satisfied, efficiency of plastic recycling, productivity, advantages resulting from selective degradation, and the like can be enhanced.

In an embodiment, when cultured together with plastic for about 15 days, *Klebsiella oxytoca* isolated from *Tenebrio molitor* larva may render a relatively high activity of degrading polyethylene terephthalate (PET), polystyrene (PS), and polyethylene (PE).

In an embodiment, when cultured together with plastic for about 30 days, *Klebsiella oxytoca* isolated from *Tenebrio molitor* larva may render a relatively higher activity of degrading polyethylene terephthalate (PET), polyvinyl chloride (PVC), and polypropylene (PP) and a high activity of degrading polystyrene (PS) and polyethylene (PE).

In an embodiment, when cultured together with plastic for about 15 days, *Escherichia fergusonii* isolated from *Tenebrio molitor* larva may render a high activity of degrading all of polyethylene terephthalate (PET), polyvinyl chloride (PVC), polystyrene (PS), polypropylene (PP), and polyethylene (PE).

In an embodiment, when cultured together with plastic for about 30 days, *Escherichia fergusonii* isolated from *Tenebrio molitor* larva may render a high activity of degrading all of polyethylene terephthalate (PET), polyvinyl chloride (PVC), polystyrene (PS), polypropylene (PP), and polyethylene (PE).

In an embodiment, when cultured together with plastic for about 15 days, *Bacillus toyonensis* reborn (Accession (deposition) Number: KACC 81043BP) isolated from *Tenebrio molitor* larva may render a relatively high activity of degrading polyethylene terephthalate (PET), polyvinyl chloride (PVC), and polyethylene (PE).

In an embodiment, when cultured together with plastic for about 30 days, *Bacillus toyonensis* reborn (Accession (deposition) Number: KACC 81043BP) isolated from *Tenebrio molitor* larva may render a relatively high activity of degrading polyethylene terephthalate (PET), polyvinyl chloride (PVC), polystyrene (PS), and polyethylene (PE) or of degrading polyethylene terephthalate (PET), polyvinyl chloride (PVC), and polyethylene (PE).

According to another aspect of the present invention, there is provided a method of degrading plastic, which includes culturing a microorganism having plastic degradation activity together with plastic-containing waste or waste plastic, wherein the microorganism having plastic degradation activity is isolated from *Tenebrio molitor* larva and is one or more selected from the group consisting of *Klebsiella oxytoca, Escherichia fergusonii,* and *Bacillus toyonensis* reborn (Accession (deposition) Number: KACC 81043BP).

In the method of degrading plastic according to the present invention, the plastic or the waste plastic may be one or more selected from polyethylene terephthalate (PET), polyvinyl chloride (PVC), polystyrene (PS), polypropylene (PP), and polyethylene (PE).

In this case, waste plastic or plastic-containing waste is treated using a combination of process parameters including the microorganism having plastic degradation activity according to one aspect of the present invention, such as a culture temperature, and a culture duration, so that it is possible to degrade a relatively large amount of specific plastic or all plastics into low-molecular-weight materials. For example, in the plastic recycling process such as a plastic pretreatment process, it is possible to selectively degrade only a desired plastic material or degrade all plastic, and accordingly, the purity and quality of recycled plastic according to a material type (property) can be enhanced.

The culture temperature may be, for example, 25 °C to 40 °C, and the culture duration may be, for example, 10 days to 60 days. When the above-described culture conditions are satisfied, efficiency of plastic recycling, productivity, advantages resulting from selective degradation, and the like can be enhanced.

The temperature and duration of the culture are not necessarily limited to the above-exemplified conditions and may be varied in various ranges depending on the type of plastic to be degraded, a combination of microorganisms having plastic degradation activity, and the like.

For example, in the method of degrading plastic according to an embodiment of the present invention, a culture temperature of the microorganism may range from 28°C to 37°C, and a culture duration thereof may range from 15 days to 30 days. When the above-described culture conditions are satisfied, efficiency of plastic recycling and productivity can be enhanced.

Another aspect of the present invention provides a method of degrading plastic, which includes reacting plastic-containing waste or waste plastic with an enzyme having plastic degradation activity, wherein the enzyme having plastic degradation activity is produced by a microorganism isolated from *Tenebrio molitor* larva and being one or more selected from the group consisting of *Klebsiella oxytoca, Escherichia fergusonii,* and *Bacillus toyonensis* reborn (Accession (deposition) Number: KACC 81043BP).

As described above, the plastic or the waste plastic may be one or more selected from polyethylene terephthalate (PET), polyvinyl chloride (PVC), polystyrene (PS), polypropylene (PP), and polyethylene (PE).

The enzyme having plastic degradation activity may be an extracellular substance or intracellular substance of the microorganism having plastic degradation activity, a protein isolated therefrom and purified, or the like, and may be produced in large quantities through genetic modification.

The method of degrading plastic according to the present invention may be applied as a pretreatment process for recycling plastic.

Another aspect of the present invention provides a culture solution of *Bacillus toyonensis* reborn (Accession (deposition) Number: KACC 81043BP) isolated from *Tenebrio molitor* larva and having plastic degradation activity.

The plastic may be one or more selected from the group consisting of polyethylene terephthalate (PET), polyvinyl chloride (PVC), polystyrene (PS), and polyethylene (PE) or the group consisting of polyethylene terephthalate (PET), polyvinyl chloride (PVC), and polyethylene (PE).

In the present invention, in order to identify a microorganism with an excellent ability to degrade plastic, a *Tenebrio molitor* subject containing the internal organs was pulverized, diluted by suspension in sterile saline, then smeared on an LB agar medium, and cultured at 28°C or 37°C for 48 hours to form colonies. Afterward, the colonies thus formed were sub-cultured to isolate a strain with an excellent ability to degrade plastic. The strain thus isolated was identified as a new strain belonging to *Bacillus toyonensis,* and the inventors of the present invention named the above-selected strain *Bacillus toyonensis* reborn and deposited the strain in the National Institute of Agricultural Sciences on January 17, 2017, and the Accession (deposition) Number of KACC 81043BP was assigned to the stain.

The *Bacillus toyonensis* reborn is a strain isolated from *Tenebrio molitor* larva and is selected by a nonpathogenic search experiment and a plastic degradation capability search experiment.

Such a *Bacillus toyonensis* reborn strain was found to be a congeneric strain having a homology of 99% or more with *Bacillus toyonensis* from the phylogenetic characteristic analysis results.

The phylogenetic characteristics were analyzed by a 16S rDNA sequence analysis and a phylogenetic tree analysis using PCR. Morphological characteristics were analyzed by gram staining, endospore staining, and scanning electron microscope (SEM) photography. Physiological characteristics were analyzed by a kit assay(API 50CHB), an antibiotic sensitivity test, and an enzymatic activity assay. In this way, the analysis of the *Bacillus toyonensis* reborn strain was carried out under the same conditions as described in Preparation Example 1.

The *Bacillus toyonensis* reborn identified by the above-described analysis methods has a high ability to degrade plastic. Therefore, when the *Bacillus toyonensis* reborn strain is used in the pretreatment process for recycling plastic and the like, efficiency of plastic recycling, productivity, advantages resulting from selective degradation, and the like can be enhanced.

Hereinafter, the present invention will be described in further detail with reference to embodiments thereof. However, it should be understood that the following embodiments disclosed herein are provided to aid understanding the present invention, and are not intended to limit the scope of the present invention.

### Preparation Example 1. Isolation, selection, and identification of microorganism of Tenebrio molitor larva

### (1) Isolation of microorganism of Tenebrio molitor larva

About 40 *Tenebrio molitor* larvae (total weight: 5 g) fed only expanded polystyrene (EPS) and polyvinyl chloride (PVC) for 2 weeks were put into 50 ml of saline (0.85% NaCl), and pulverized with a pulverizer for about 30 seconds to obtain a pulverized *Tenebrio molitor* larva solution. Then, the pulverized *Tenebrio molitor* larva solution was diluted with saline at varying dilution factors (10⁻⁴, 10⁻⁵, 10⁻⁶, 10⁻⁷) to obtain four diluted *Tenebrio molitor* larva solutions.

Afterward, each of the diluted *Tenebrio molitor* larva solutions was inoculated on an LB agar medium and an Difco nutrient broth to prepare a total of 16 inoculation plates, and each plate was cultured at 28°C or 37°C for 2 days to obtain culture plates in which multiple colonies were formed.

The LB agar medium was prepared by adding 10 g of LB broth and 10 g of agar to 500 ml of distilled water, followed by mixing and sterilization in a sterilizer for 40 minutes. In addition, the Difco nutrient broth was prepared by adding 1.5 g of Difco nutrient broth, 12.5 g of yeast extract, and 10 g of agar to 500 ml of distilled water, followed by mixing and sterilization in a sterilizer for 40 minutes.

Afterward, individual colonies were taken from multiple colonies formed in the culture plate, smeared by streaking on an LB agar medium and an Difco nutrient broth, and then cultured under the same culture conditions as the corresponding multiple colonies for 1 day to obtain a total of 28 colonies of a single species through isolation.

Before selection, the microorganisms of 28 single-species colonies thus isolated were primarily sorted based on color, smoothness of shape, and tail motility to obtain a total of 19 isolated single-species colonies. In the primary sorting prior to selection, the morphology of each sample was visually confirmed from the microscopic observation result, and a total of 9 species of strains judged to be similar species were excluded. Among 19 isolated single-species colonies, 7 colonies had been cultured at 28°C, and 12 colonies had been cultured at 37°C.

### (2) Search for ability of microorganism isolated from Tenebrio molitor larva to degrade plastic (solid medium)

Each of 19 single-species colonies obtained as above was smeared by streaking on a carbon-free basal agar medium (CFBAM) at 10⁸ cfu/ml to prepare a CFBAM plate. The CFBAM was prepared by adding 0.7 g of KH₂PO₄, 0.7 g of K₂HPO₄, 0.7 g of MgSO₄·H₂O, 1.0 g of NH₄NO₃, 0.005 g of NaCl, 0.002 g of FeSO₄·H₂O, 0.002g of ZnSO₄·H₂O, 0.001 g of MnSO₄·H₂O, and 15 g of agar to 1000 ml of distilled water, followed by mixing and sterilization in a sterilizer for 40 minutes.

Afterward, the CFBAM plate was divided into four regions, and a polystyrene (PS) film, a polyvinyl chloride (PVC) film, a polypropylene (PP) film, and a polyethylene terephthalate (PET) film, all of which had the same thickness and size and a weight of 2 g, were installed respectively in the four regions. Since the CFBAM has no carbon sources, the isolated microorganisms cannot survive unless they degrade the plastic film, which can be used as an index for evaluating the ability to degrade plastic.

However, since microorganisms that degrade agar to survive may sparsely exist, to confirm this, a CFBAM plate, in which single colonies were smeared on a CFBAM, and plastic films were not additionally installed, was prepared as a control group.

Afterward, the plastic-film-installed CFBAM plate and the CFBAM plate without plastic films installed were cultured at 28°C or 37°C for 28 days. Microorganisms that grow around the plastic film but do not grow in the corresponding control can be considered to have plastic degradation activity.

After the 28-day culture, it was confirmed through visual observation that 10 species of microorganisms among the total of 19 species of microorganisms survived at the edge of film and produced colonies. Detailed results thereof are shown in the following Table 1.

**[Table 1]**

| Classification | Culture temperature (°C) | PET | PP | PVC | PS | Plastic degradation activity search results | Control |
|---|---|---|---|---|---|---|---|
| Experimental Example 1 | 28 | Δ | X | ○ | ○ | Fair | X |
| Experimental Example 2 | 28 | Δ | Δ | ○ | ⊚ | Very suitable | ○ |
| Experimental Example 3 | 28 | ○ | ○ | ○ | ⊚ | Very suitable | X |
| Experimental Example 4 | 28 | X | X | ⊚ | ○ | Fair | X |
| Experimental Example 5 | 37 | X | X | Δ | ⊚ | Fair | X |
| Experimental Example 6 | 37 | X | X | ○ | X | Fair | X |
| Experimental Example 7 | 37 | X | Δ | ○ | ○ | Fair | X |
| Experimental Example 8 | 37 | ○ | X | ⊚ | ○ | Very suitable | X |
| Experimental Example 9 | 37 | ⊚ | X | ⊚ | ⊚ | Very suitable | X |
| Experimental Example 10 | 37 | X | X | ○ | X | Fair | X |

According to Table 1, the degree at which the microorganisms formed colonies on the plastic film was evaluated through visual observation. In this case, "⊚" indicates a case in which a large number of microbial colonies were formed on the entire surface of the plastic film and around the plastic film, "○" indicates a case in which microbial colonies were formed on all four sides around the plastic film, "Δ" indicates a case in which microbial colonies were formed only on one side or part of the plane around the plastic film, and "×" indicates a case in which microorganisms did not grow around the plastic film.

In the evaluation of four samples, PET, PP, PVC, and PS, based on the above results, "⊚" was given 3 points, "○" was given 2 points, "Δ" was given 1 point, and "×" was given 0 points, and the scores of each sample were added up. The resulting value of 7 points or more was evaluated as "very suitable" as a search result of the ability to degrade plastic, less than 7 points was evaluated as "fair", and 0 points was evaluated as "unsuitable".

### (3) Final selection and identification of microorganism

The DNA of the microorganisms described in Table 1 was extracted, amplified, and then subjected to a 16S rDNA sequence analysis to identify the species of microorganism. Among 10 species of microorganisms described in Table 1, highly pathogenic microorganisms were excluded from the selection.

Each of 10 species of isolated strains described in Table 1 was cultured to obtain a single colony, and a strain suspension was then prepared. The strain suspension was subjected to genomic isolation using a microorganism identification kit (Trade name: Wizard® genomic DNA purification kit, Manufacturer: Promega Corporation), and the result thereof was input into a NCBI database to analyze the base sequence homology of isolated strains. Results thereof are shown in Table 2.

Through the identification of strains, the microorganisms of Experimental Example 2, Experimental Example 3, and Experimental Example 9 were selected as final strains excluding the microorganisms which were not suitable for selection.

**[Table 2]**

| Classification | Culture temperature (°C) | Species name of identified strain | Homology strain (based on 16S rDNA) | Whether to select or not |
|---|---|---|---|---|
| Experimental Example 1 | 28 | *Citrobacter freundii* | *Citrobacter freundii* | Unsuitable |
| Experimental Example 2 | 28 | *Klebsiella oxytoca* | *Klebsiella oxytoca* strain ATCC 13182 | Selected |
| Experimental Example 3 | 28 | *Escherichia fergusonii* | *Escherichia fergusonii* strain ATCC 35469 | Selected |
| Experimental Example 4 | 28 | *Salmonella bongori, Citrobacter sedlakii* | *Salmonella bongori, Citrobacter sedlakii* | Unsuitable |
| Experimental Example 5 | 37 | *Enterococcus avium* | *Enterococcus avium* | Unsuitable |
| Experimental Example 6 | 37 | *Citrobacter freundii* | *Citrobacter freundii* | Unsuitable |
| Experimental Example 7 | 37 | *Enterococcus avium* | *Enterococcus avium* | Unsuitable |
| Experimental Example 8 | 37 | *Escherichia fergusonii, Shigella flexneri* | *Escherichia fergusonii, Shigella flexneri* | Unsuitable |
| Experimental Example 9 | 37 | *Bacillus toyonensis* | *Bacillus toyonensis* strain BCT-7112 | Selected |
| Experimental Example 10 | 37 | *Escherichia fergusonii, Shigella flexneri* | *Escherichia fergusonii, Shigella flexneri* | Unsuitable |

### (4) Naming and deposit of strain of selected microorganism

The inventors of the present invention named the selected strain of microorganism of Experimental Example 7 *Bacillus toyonensis* reborn. In addition, the inventors of the present invention deposited the *Bacillus toyonensis* reborn by patent deposit in an international depositary authority, National Institute of Agricultural Sciences (address: 166, Nongsaengmyeong-ro, lseo-myeon, Wanju-gun, Jeollabuk-do, Korea), on January 17, 2017, and the Accession (deposition) Number of KACC 81043BP was assigned to the strain. The deposit of microorganisms was implemented based on the Rules of the Budapest Treaty on the International Recognition of the Deposit of Microorganisms for the Purposes of Patent Procedure.

### <Evaluation method>

### Evaluation of plastic degradation activity of selected microorganism on liquid medium (liquid property medium)

In the recycling process of waste plastic, the pretreatment process, in which some plastics are degraded using the above-selected microorganisms or enzymes produced by the selected microorganisms, is commonly carried out under a liquid property condition. Therefore, the plastic degradation activities of the finally selected microorganisms were determined using a liquid property medium under conditions similar to those of the pretreatment process in the recycling process of waste plastic.

Specifically, 0.7 g of KH₂PO₄, 0.7 g of K₂HPO₄, 0.7g of MgSO₄·H₂O, 1.0 g of NH₄NO₃, 0.005 g of NaCl, 0.002 g of FeSO₄·H₂O, 0.002 g of ZnSO₄·H₂O, and 0.001 g of MnSO₄·H₂O were added to 1000 ml of distilled water and mixed, and the resultant mixture was then sterilized in a sterilizer for 40 minutes to prepare a liquid carbon-free basal medium (LCFBM). Afterward, 40 ml of the LCFBM and 500 mg of each of plastic particles (PET, PVC, PS, PP, and PE) were put into an Erlenmeyer flask, and each of 3 species of the selected microorganisms was inoculated thereto at a concentration of 10⁸ CFU/ml and cultured using a shaking incubator at 32 °C and 160 rpm for 23 days. Meanwhile, only an LCFBM and each of plastic particles were put into an Erlenmeyer flask and cultured using a shaking incubator at 32 °C and 160 rpm for 23 days to prepare a control.

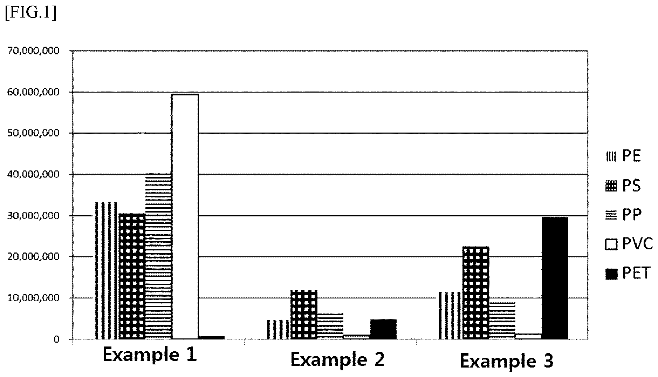

The colony count of individual microorganisms, the amount of residual plastic, the amount of degraded plastic, and the like were measured, and results thereof are shown in the following Table 3 to Table 5. Also, the results of Table 3 were plotted on a graph of FIG. 1.

More specifically, on the 20^{th} day of microorganism culture, a culture solution was sampled, a remaining plastic sample was weighed, and a decrease in weight of the plastic sample was measured.

**[Table 3]**

| (cfu/ml) | Strain | PE | PS | PP | PVC | PET |
|---|---|---|---|---|---|---|
| Example 1 | *Klebsiella oxytoca* (Experimental Example 2) | 33200000 | 30500000 | 40800000 | 59400000 | 700000 |
| Example 2 | *Escherichia fergusonii* (Experimental Example 3) | 4600000 | 12000000 | 6500000 | 900000 | 4900000 |
| Example 3 | KACC 81043BP (Experimental Example 7) | 11600000 | 22500000 | 8800000 | 1300000 | 29700000 |

**[Table 4]**

| Remaining amount (g) | PET | PVC | PS | PP | PE |
|---|---|---|---|---|---|
| Example 1 | 0.2046 | 0.3478 | 0.486 | 0.3806 | 0.449 |
| Example 2 | 0.259 | 0.3136 | 0.4847 | 0.4669 | 0.4697 |
| Example 3 | 0.2133 | 0.3503 | 0.4536 | 0.4625 | 0.4118 |
| Control | 0.4733 | 0.4867 | 0.4929 | 0.489 | 0.4952 |

**[Table 5]**

| Degradation amount (g) | PET | PVC | PS | PP | PE |
|---|---|---|---|---|---|
| Example 1 | 0.2954 | 0.1522 | 0.014 | 0.1194 | 0.051 |
| Example 2 | 0.241 | 0.1864 | 0.0153 | 0.0331 | 0.0303 |
| Example 3 | 0.2867 | 0.1497 | 0.0464 | 0.0375 | 0.0882 |
| Control | 0.0267 | 0.0133 | 0.0071 | 0.011 | 0.0048 |

| | | | | | |
|---|---|---|---|---|---|
| PET: Polyethylene terephthalate * PVC: Polyvinyl chloride * PS: Polystyrene * PP: Polypropylene * PE: Polyethylene | | | | | |

As can be seen in Tables 3 to 5, on the 23^{rd} day of microorganism culture at 32 °C and 160 rpm, the selected strains, that is, *Klebsiella oxytoca* had a high degradation activity especially for PET, PVC, and PP, *Escherichia fergusonii* had a high degradation activity especially for PET and PVC, and *Bacillus toyonensis* had a high degradation activity especially for PET and PVC.

From these results, it can be seen that when microorganisms are cultured under conditions of 32 °C, 160 rpm, and 23 days, the selected strains, that is, *Klebsiella oxytoca* can exhibit an improved ability to degrade PET, PVC, and PP, *Escherichia fergusonii* can exhibit an improved ability to degrade PET and PVC, and *Bacillus toyonensis* can exhibit an improved ability to degrade PET and PVC.

Generally, in the plastic recycling, individual plastic materials are not compatible with each other, so all other plastic materials other than one plastic material intended for recycling need to be eliminated.

If a digester (for example, with a capacity of 10 tons) for degrading and eliminating other kinds of plastic while leaving a specific plastic in 3 tons of mixed waste plastic per day is used, there are 2 wt% to 5 wt% foreign substances (60 kg to 150 kg of other waste plastics to be degraded and the like) in 3 tons of mixed waste plastic having undergone a pretreatment process.

The digester space remaining after 3 tons of mixed waste plastic is introduced to a digester may be charged with 10⁸ cfu/mL of the microorganism isolated from *Tenebrio molitor* larva according to the present invention or 7 tons of a culture solution including the microorganism. In the case of 1 mL of a strain-containing culture solution, 0.612 to 1.53 mg/per day of other waste plastic materials need to be degraded into water-soluble metabolites to completely eliminate foreign substances from mixed waste plastic.

As can be seen from the above experimental results, the *Bacillus Toyonensis* (KACC 81043BP) strain according to the present invention degrades 286.7 mg of PET and 149.7 mg of PVC per day. From this fact, it can be seen that the microorganism isolated from *Tenebrio molitor* larva according to the present invention is highly effective for selectively eliminating a PET label in a container made of PP, PE, or the like during the recycling of PP, PE, or the like.

In addition, the culture solutions sampled on the 15^{th} day of microorganism culture in the same manner as described above were filtered, and the filtered solutions were analyzed through gas chromatography to check whether the content of a low-molecular-weight material was increased. As a result of the gas chromatography analysis, the content of a low-molecular-weight material was significantly increased in most culture solutions.

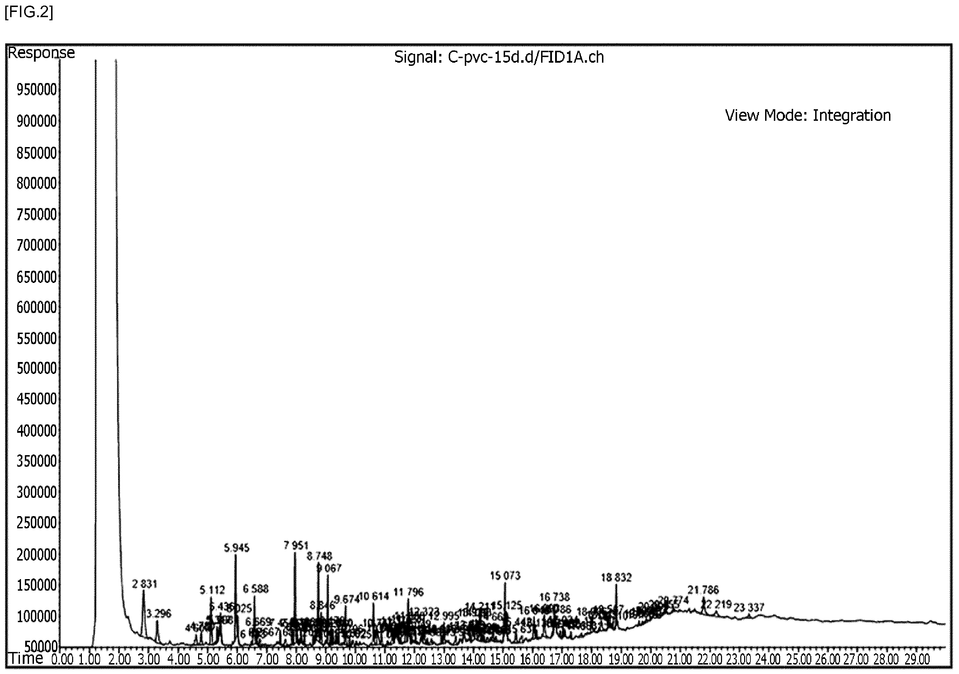

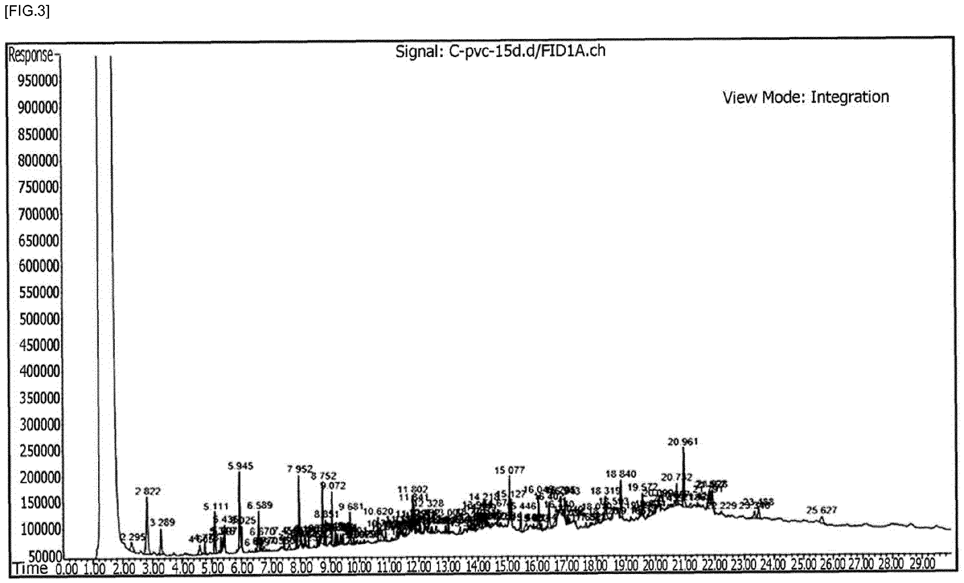

FIG. 2 shows the gas chromatography analysis result of a culture solution of a control after 15 days of culture in a polyvinyl chloride (PVC) degradation experiment using a liquid medium. FIG. 3 shows the gas chromatography analysis result of a culture solution of a selected strain, *Escherichia fergusonii,* after 15 days of culture in a PVC degradation experiment using a liquid medium.

As shown in FIG. 2 and FIG. 3, in the culture solution of *Escherichia fergusonii,* the area of a peak corresponding to a low-molecular-weight material having a short retention time was significantly increased compared to the culture solution of a control.

While the present invention has been described above with reference to the exemplary embodiments, the present invention is not limited thereto. It is clear that various modifications may be made without departing from the scope and spirit of the present invention. Therefore, it will be understood that the scope of the present invention encompasses all embodiments included in the scope of the claims appended in the present invention.

### (Accession (deposition) Number)

Name of Depository: National Institute of Agricultural Sciences
Accession (deposition) Number: KACC 81043BP
Deposit Date: 20170117

## Claims

1. A microorganism isolated from *Tenebrio molitor* larva, selected from *Klebsiella oxytoca, Escherichia fergusonii,* and *Bacillus toyonensis* reborn (Accession (deposition) Number: KACC 81043BP), and having plastic degradation activity.

2. The microorganism of claim 1, wherein the plastic is one or more selected from the group consisting of polyethylene terephthalate (PET), polyvinyl chloride (PVC), polystyrene (PS), polypropylene (PP), and polyethylene (PE).

3. A method of degrading plastic, comprising culturing a microorganism having plastic degradation activity together with plastic-containing waste or waste plastic,
wherein the microorganism having plastic degradation activity is isolated from *Tenebrio molitor* larva and is one or more selected from the group consisting of *Klebsiella oxytoca, Escherichia fergusonii,* and *Bacillus toyonensis* reborn (Accession (deposition) Number: KACC 81043BP).

4. The method of claim 1, wherein the plastic or the waste plastic is one or more selected from the group consisting of polyethylene terephthalate (PET), polyvinyl chloride (PVC), polystyrene (PS), polypropylene (PP), and polyethylene (PE).

5. The method of claim 4, wherein a temperature and duration of the culture of a microorganism is 25°C to 40°C and 10 days to 60 days, respectively.

6. A method of degrading plastic, comprising reacting plastic-containing waste or waste plastic with an enzyme having plastic degradation activity,
wherein the enzyme having plastic degradation activity is produced by a microorganism isolated from *Tenebrio molitor* larva and being one or more selected from the group consisting of *Klebsiella oxytoca, Escherichia fergusonii,* and *Bacillus toyonensis* reborn (Accession (deposition) Number: KACC 81043BP).

7. The method of claim 6, wherein the plastic or the waste plastic is one or more selected from the group consisting of polyethylene terephthalate (PET), polyvinyl chloride (PVC), polystyrene (PS), polypropylene (PP), and polyethylene (PE).

8. A culture solution of *Bacillus toyonensis* reborn (Accession (deposition) Number: KACC 81043BP) isolated from *Tenebrio molitor* larva and having plastic degradation activity.

9. The culture solution of claim 8, wherein the plastic is one or more selected from the group consisting of polyethylene terephthalate (PET), polyvinyl chloride (PVC), polystyrene (PS), polypropylene (PP), and polyethylene (PE).
